# EUROPÄISCHE PATENTSCHRIFT

(11) **EP 1 324 728 B1**
(45) Veröffentlichungstag und Bekanntmachung des Hinweises auf die Patenterteilung: **06.04.2005**
(21) Anmeldenummer: 00966161.2
(22) Anmeldetag: 11.10.2000
(51) Int. Cl.: A61F 5/01

(54) **DIAGNOSTIK- UND REHABILITATIONSMATTE**
DIAGNOSTIC AND REHABILITATION MAT
NATTE DE DIAGNOSTIC ET DE READAPTATION

(43) Veröffentlichungstag der Anmeldung: 09.07.2003
(73) Patentinhaber: KS ITALIA di Ambrosone Mario e C. s.a.s., 83100 Avellino (IT)
(72) Erfinder: FUSCO, Maria, Antonietta, I-83100 Avellino (IT)
(74) Vertreter: Menges, Rolf, Dipl.-Ing.
(86) Internationale Anmeldenummer: PCT/EP2000/010027
(87) Internationale Veröffentlichungsnummer: WO 2002/030342

(56) Entgegenhaltungen:
- EP-A- 0 917 835
- DE-A- 3 905 989
- US-A- 4 823 799

## Beschreibung

### Technisches Gebiet

Die Erfindung betrifft eine Matte der im Oberbegriff des Patentanspruchs 1 angegebenen Art.

### Stand der Technik

Durch geeignete Stimulierung der Fuß-Nervenrezeptoren kann eine Wiederherstellung der Vaskulärfunktionen des Fußes erreicht werden, indem der venöse und der lymphatische Blutkreislauf der unteren Gliedmaßen verbessert wird. Ebenso wird eine verbesserte Wiederherstellung des Gleichgewichts und der Knochen- und Gelenkstrukturen des gesamten Organismus, insbesondere nach chirurgischen Eingriffen und/oder Traumen, ermöglicht.

Durch Destabilisierung der Fußauflage und Stimulierung der Fuß-Nervenrezeptoren ist es möglich, die Vestibulärfunktion (ital.: funzione vestibolare) zu diagnostischen und Rehabilitationszwecken zu isolieren.

Im weitesten Sinne werden bei der Erfindung Reflexzonen des Fußes in Betracht gezogen, wie bei einer Einlegesohle, welche aus der EP-A 0 917 835 bekannt ist. Diese bekannte Einlegesohle wird in einen Schuh eingelegt und weist elastische Wölbungen im Bereich der Proprio- und Barozeptoren sowie der Reflexzonenpunkte auf der Fußsohle gemäß der traditionellen chinesischen Medizin auf. Bekanntlich besteht die Fußreflexzonentherapie in einer Stimulierung bestimmter Punkte auf der Fußsohle, die im wesentlichen den Muskeleinbettungen der sogenannten Implizit-Muskeln zur Aufrechterhaltung der Sohlenwölbung entsprechen, durch die man die allgemeine Physiologie des menschlichen Körpers zu therapeutischen Zwecken beeinflussen kann. Insbesondere kann man sogenannte Anomalien der aufrechten Haltung therapeutisch korrigieren. Die Einlegesohle umfasst mehrere Wölbungen, die sich im Bereich der Proprio- und Barozeptoren sowie der Reflexzonenpunkte der Fußsohle befinden. Diese Proprio- und Barozeptoren von oberflächigem, artikuliertem und tiefem Typ sind im wesentlichen in den Bereichen der Muskeleinbettungen angelegt. Um die Wölbungen zwischen der ersten und zweiten Schicht in den Bereichen der Proprio- und Barozeptoren sowie der Reflexzonenpunkte der Fußsohle zu bilden, ist die Einlegesohle mit mehreren Alveolen ausgestattet, in deren Bereich zwischen der ersten und zweiten Schicht kein diese Schichten zusammenhaltender Klebstoff vorhanden ist. Der Umfang der Alveolen wird durch entsprechende Linearnähte definiert und verstärkt. Die Alveolen sind jeweils derart gestaltet, dass sie mit elastischen Einzelpartikeln im wesentlichen unregelmäßiger Form, facettiert und kantig und aus im wesentlichen nicht allergenem Gummi verfüllt werden können, um die Wölbungen zu bilden. Zu diesem Zweck ist jede Alveole mit einer Öffnung ausgestattet, die sich in der Nähe der entsprechenden Lineamaht befindet, um die Füllung der Alveolen mittels einer Kanüle oder eines analogen Instruments zu ermöglichen. Die Öffnungen können sofort nach Beendigung des Füllvorgangs verschlossen werden. Die Partikel können dadurch hergestellt werden, dass die gleiche Para-Gummifolie, die zur Konfektionierung einer ersten Schicht der Einlegesohle verwendet wird, in Würfel geschnitten wird, die jeweils eine Querabmessung von etwa 1 mm aufweisen. Zur Bildung einer zweiten Schicht der Einlegesohle wird ein berührungsweiches Material verwendet, das für den Kontakt mit der Fußsohle geeignet ist. Insbesondere wird Wildleder bzw. Alcantara verwendet. Die Dicke jeder Wölbung, die durch Füllen mit den Partikeln und durch die natürliche Elastizität von z.B. Alcantara und von Gummi entsteht, beträgt etwa 3 mm. Form, Dicke und Elastizität jeder Wölbung werden für eine korrekte Reflexzonenbeanspruchung der Proprio- und Barozeptoren der Fußsohle im voraus festgelegt. Insbesondere die Füllung der Alveolen wird auf der Grundlage therapeutischer Vorgaben bezogen auf den die Sohle tragenden Patienten durchgeführt. Jede Sohle weist sieben elastische Wölbungen auf, die je nach ihrer Lage unterschiedlich geformt sind und zu den entsprechenden Bereichen der Proprio- und Barozeptoren der Fußsohle passen, insbesondere eine erste elastische Wölbung in Höhe des Adduktors der großen Zehe, eine zweite Wölbung für den kurzen Flexor der großen Zehe, eine dritte Wölbung für den kurzen Flexor der kleinen Zehe, eine vierte Wölbung für den Adduktor der kleinen Zehe, eine fünfte Wölbung für den Dorsalkeil, eine sechste Wölbung für den Plantarkeil und eine siebte Wölbung für den Adduktor der großen Zehe. Die bekannte Sohle kann Variationen unterliegen. So können z.B. die einzelnen Partikel zur Füllung der Alveolen durch anderes Material ersetzt werden, sei es gallertartig oder pulverförmig oder jedenfalls weich, und das Material kann natürlichen oder synthetischen Ursprungs sein. Insbesondere ist es möglich, synthetischen Gummi, zum Beispiel Silikongummi, als Ersatz für Paragummi zu verwenden. Die Reflexzonenpunkte auf der Fußsohle werden gemäß der traditionellen chinesischen Medizin bestimmt. Die Alveolen ergeben durch Füllen mit elastischen Partikeln elastische Wölbungen, deren Form, Dicke und Elastizität so gewählt werden, dass sich die korrekte reflexzonentherapeutische Reizung in den Reflexzonenpunkten auf der Fußsohle gemäß der traditionellen chinesischen Medizin ergibt.

### Darstellung der Erfindung

Aufgabe der Erfindung ist es, ein geeignetes Mittel für die vorgenannten vielfältigen Tätigkeiten zur Verfügung zu stellen, das leicht anzuwenden ist, eine lange Lebensdauer hat und die eingangs erwähnten Anwendungsfunktionen gewährleistet.

Diese Aufgabe wird durch eine Matte nach dem Oberbegriff des Patentanspruchs 1 mit den im kennzeichnenden Teil desselben angegebenen Merkmalen gelöst.

Die erfindungsgemäße Matte hat eine untere, mit dem Boden in Berührung kommende, aus natürlichem, atoxischem und nichtallergenem Gummimaterial bestehende Schicht sowie eine zweite, obere, ebenfalls aus natürlichem, atoxischem und nichtallergenem Gummimaterial bestehende Schicht, die mit den Füßen der Patienten in Berührung kommt und vorbestimmte Biegsamkeits- und Elastizitätsmerkmale aufweist. Die erste und die zweite Schicht bilden eine Füllkammer unterschiedlicher oder veränderlicher Höhe, die mit elastischem Material gefüllt ist.

Die Matte nach der Erfindung ermöglicht die Diagnose und die Rehabilitation oder Stabilisierung bei Gleichgewichtsstörungen sowie die Knochen-, Gelenk- und Gefäßrehabilitation oder -stabilisierung der Patienten. Die Matte besteht aus Materialien, die eine elastische Kraft erzeugen, welche dem Druck gleicht, dem sie ausgesetzt werden. Hierdurch bewirkt sie eine Täuschung oder Störung der Nervenendigungen der Fußsohle, insbesondere der sich rasch anpassenden Propriozeptoren, welche einen Richtungsvektor der Stimulierung und ein Depolarisierungsintervall benötigen. Die Matte stellt ein unersetzliches Instrument zur Diagnose von peripheren und/oder zentralen Vestibulopathien dar, wobei durch die Verwendung der Matte die Kontrolle durch den Fuß verloren geht. Ein Patient, der aufrecht und mit geschlossenen Augen auf der Matte steht, ist gezwungen, sich hinsichtlich der Kontrolle seiner Stellung ausschließlich auf die Informationen durch das Labyrinth zu verlassen. Auf diese Weise erhält man die funktionelle Neuprogrammierung der Vestibularinformationen.

Eine solche Matte ermöglicht sowohl das Gehen auf der Stelle als auch das Ausführen von einigen Schritten und ist für diagnostische, Rehabilitations- und Stabilisierungszwecke verwendbar.

Die bei der erfindungsgemäßen Matte verwendete Füllung kann sowohl aus natürlichen als auch synthetischen Stoffen unterschiedlicher Konsistenz oder einer entsprechenden Mischung bestehen. Diese Stoffe können in Gestalt von einzelnen elastischen Teilchen von im wesentlichen unregelmäßiger Form vorliegen und/oder eine pulverförmige, feste oder gallertartige Konsistenz aufweisen. Letztere können in unterschiedlichen Prozentsätzen elastischen Einzelteilchen aus natürlichem Paragummi beigemischt sein oder statt der elastische Einzelteilchen aus natürlichem Paragummi eingesetzt werden.

### Kurze Beschreibung der Zeichnungen

Ausführungsbeispiele der Erfindung werden im folgenden Anhand der Zeichnung näher beschrieben.

Es zeigt:
- Fig. 1: eine Ausführungsform der Matte nach der Erfindung in einer Teildraufsicht, und
- Fig. 2: eine Längsschnittansicht der Matte nach Fig. 1.

### Bester Weg zur Ausführung der Erfindung

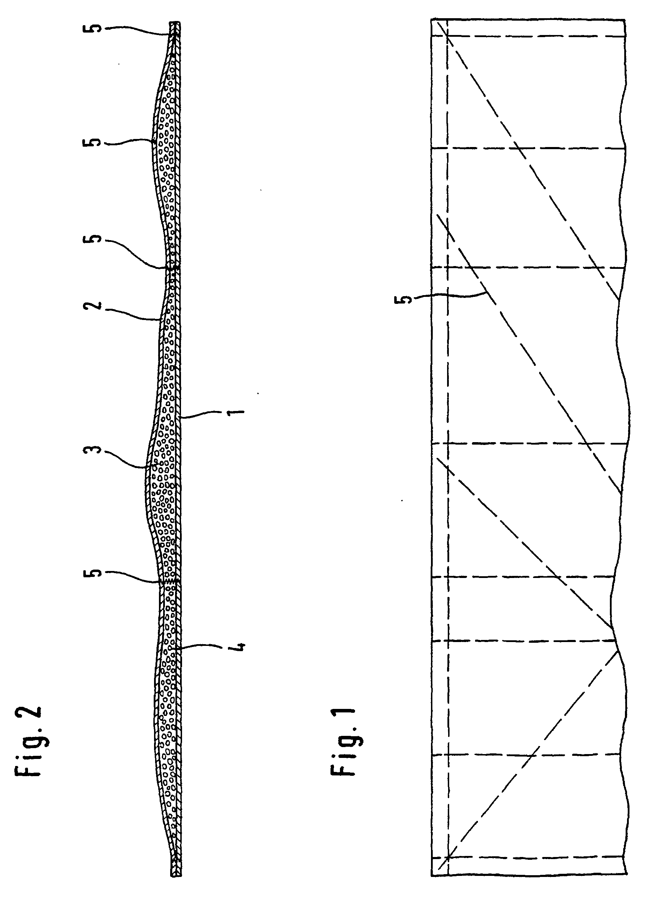

Die Fig. 1 und 2 zeigen in einer Teildraufsicht bzw. in einer Längsschnittansicht eine Matte, die aus einer ersten, unteren, mit dem Boden in Berührung kommenden Schicht 1 aus natürlichem, atoxischem und nichtallergenem Paragummi, einer zweiten, oberen Schicht 2, gleichfalls aus natürlichem Paragummi mit geradlinigen Nähten 5 an der Oberfläche, die mit Baumwollfäden in Längsrichtung der Matte und/oder Querrichtung der Matte und/oder in schräger Richtung zur Matte ausgeführt sind. Die erste Schicht 1 und die zweite Schicht 2 bilden eine Füllkammer 3 von unterschiedlicher oder veränderbarer Höhe, die je nach Bedarf mit elastischem Material 4 in Gestalt von einzelnen elastischen Teilchen von im wesentlichen unregelmäßiger Form angefüllt ist. Das Material 4 kann ein Gummimaterial sein. Die einzelnen elastischen Teilchen können mit einzelnen elastischen Teilchen aus anderen natürlichen und/oder synthetischen Stoffen vermischt sein, die eine Stimulation der Rezeptoren der Fußsohle ermöglichen, beispielsweise wie es in der eingangs erwähnten EP-A 0 917 935 beschrieben ist, um verschiedene Ziele zu erreichen:
1) Reaktivierung der physiologischen Vaskulärfunktion des Fußes;
2) Optimierung des Knochen- und Gelenkstatus des gesamten Organismus sowohl bei der posttraumatischen als auch bei postoperativen Knochen- und Gelenkrehabilitation oder - stabilisierung; und
3) Isolierung der Vestibulärfunktion durch Destabilisierung der Fußauflage.

An der oben bezeichneten Matte, der sowohl die Diagnose und die Rehabilitation von Gleichgewichtsstörungen als auch die Knochen-, Gelenk- und Gefäßrehabilitation der Patienten ermöglicht, kann ein einschlägiger Fachmann zur Erfüllung weiterer wechselnder Anforderungen zahlreiche weitere Änderungen vornehmen, die jedoch alle unter den Schutzumfang der Erfindung fallen, wie er in den beigefügten Ansprüchen festgelegt ist.

Auch die Höhe, die Länge, die Breite und die gesamte Form der Matte können im Rahmen der vorliegenden Erfindung verändert werden.

Es können auch z.B. die Nähte 5 beide Schichten 1 und 2 mehr oder weniger eng zusammenhalten oder auch nur die Schicht 2 durchsetzen.

## Patentansprüche

1. Matte zur Diagnose und Rehabilitation bei Störungen des Gleichgewichts sowie zur Knochen-, Gelenk- und Gefäßrehabilitation oder -stabilisierung, **gekennzeichnet durch** eine erste, untere, mit dem Boden in Berührung zu bringende Schicht (1) aus im wesentlichen nichtallergenem Gummimaterial und **durch** eine zweite, obere, gleichfalls aus atoxischem, nichtallergenem Gummimaterial bestehende, für die Berührung mit dem Fuß geeignete Schicht (2), wobei die erste und die zweite Schicht (1, 2) eine Füllkammer (3) unterschiedlicher oder veränderbarer Höhe bilden, die mit elastischem Material (4) gefüllt ist.

2. Matte nach Anspruch 1, **dadurch gekennzeichnet, dass** die obere, mit den Füßen in Berührung stehende Schicht (1) geradlinige, in Längsrichtung und/oder in Querrichtung der Matte und/oder in schräger Richtung zur Matte verlaufende Nähte (5) aufweist.

3. Matte nach Anspruch 1 oder 2, **dadurch gekennzeichnet, dass** das im wesentlichen nichtallergene Gummimaterial aus Naturgummi, insbesondere aus Paragummi mit vorbestimmter Biegsamkeit und Elastizität besteht.

4. Matte nach Anspruch 1 oder 2, **dadurch gekennzeichnet, dass** das im wesentlichen nichtallergene Gummimaterial ein synthetischer Gummi ist, beispielsweise Silikongummi oder eine Gummiart jeder anderen Zusammensetzung.

5. Matte nach Anspruch 1 oder 2, **dadurch gekennzeichnet, dass** das im wesentlichen nichtallergene Gummimaterial eine Mischung aus synthetischem Gummi und natürlichem Paragummi ist.

6. Matte nach Anspruch 1 oder 2, **dadurch gekennzeichnet, dass** das im wesentlichen nichtallergene Gummimaterial ein natürliches oder synthetisches Gewebe ist.

7. Matte nach Anspruch 1 oder 2, **dadurch gekennzeichnet, dass** das im wesentlichen nichtallergene Gummimaterial eine Mischung aus einem natürlichen oder synthetischen Gewebe und natürlichem Paragummi ist.

8. Matte nach einem der Ansprüche 1 bis 7, **dadurch gekennzeichnet, daß** das elastische Material der Füllung der Füllkammer (3) zwischen der ersten und der zweiten Schicht (1, 2) in Gestalt von einzelnen elastischen Teilchen von im wesentlichen unregelmäßiger Form verliegt oder durch ein anderes gallertartiges oder pulverförmiges Material oder auch durch ein Material jeder sonstigen elastischen Konsistenz gebildet ist, oder dieses auch mit den elastischen Teilchen in unterschiedlichem und zweckmäßigem Prozentsatz vermischt ist.

9. Matte nach einem der Ansprüche 1 bis 8, **dadurch gekennzeichnet, daß** das elastische Material aus einem natürlichen oder synthetischen Stoff, beispielsweise Paragummi oder Silikongummi, besteht oder eine Mischung aus unterschiedlichen Stoffen ist.

## Claims

1. A diagnostic and rehabilitation mat for cases of balance disturbance and for the rehabilitation or stabilization of bones, joints and vessels, **characterized by** a first, lower layer (1) for floor contact, comprised of a substantially non-allergenic rubber material, and by a second, upper layer (2) suitable for contact with the foot and likewise comprised of a non-toxic, non-allergenic rubber material, said first and said second layers (1, 2) forming a filler chamber (3) of a different or variable height, the chamber being filled with elastic material (4).

2. The mat according to claim 1, **characterized in that** the upper layer (1) in contact with the feet has straight seams (5) running longitudinally and/or transversely and/or diagonally to the mat.

3. The mat according to claim 1 or 2, **characterized in that** the substantially non-allergenic rubber material consists of natural rubber, particularly para rubber with predetermined flexibility and elasticity.

4. The mat according to claim 1 or 2, **characterized in that** the substantially non-allergenic rubber material is a synthetic rubber such as silicone rubber or a type of rubber having any other composition.

5. The mat according to claim 1 or 2, **characterized in that** the substantially non-allergenic rubber material is a mixture of synthetic rubber and natural para rubber.

6. The mat according to claim 1 or 2, **characterized in that** the substantially non-allergenic rubber material is a natural or synthetic fabric.

7. The mat according to claim 1 or 2, **characterized in that** the substantially non-allergenic rubber material is a mixture of a natural or synthetic fabric and natural para rubber.

8. The mat according to any one of claims 1 to 7, **characterized in that** the elastic material of the filling of the filler chamber (3) between the first and the second layers (1, 2) is provided in the form of individual elastic particles of substantially irregular shape, or it is formed by another gelatinous or powdery material or by a material of any other elastic consistency, or said material is also mixed with the elastic particles at a different and suitable percentage.

9. The mat according to any one of claims 1 to 8, **characterized in that** the elastic material consists of a natural or synthetic substance such as para rubber or silicone rubber, or it is a mixture of different substances.

## Revendications

1. Tapis pour le diagnostic et la rééducation en cas de troubles de l'équilibre ainsi que pour la rééducation ou la stabilisation des os, des articulations et des vaisseaux, **caractérisé par** une première couche (1) inférieure, à placer en contact avec le sol, en caoutchouc sensiblement non allergénique, et par une seconde couche (2) supérieure adaptée pour le contact avec le pied, en caoutchouc atoxique, non allergénique, la première et la seconde couche (1, 2) formant une chambre (3) rembourrée, de hauteur différente ou variable, qui est remplie d'un matériau élastique (4).

2. Tapis selon la revendication 1, **caractérisé en ce que** la couche (1) supérieure en contact avec les pieds présente des coutures (5) droites, en direction longitudinale et/ou en direction transversale du tapis et/ou en direction oblique par rapport au tapis.

3. Tapis selon l'une des revendications 1 ou 2, **caractérisé en ce que** le caoutchouc sensiblement non allergénique est constitué de caoutchouc naturel, en particulier de caoutchouc Para doté d'une flexibilité et d'une élasticité prédéterminées.

4. Tapis selon l'une des revendications 1 ou 2, **caractérisé en ce que** le caoutchouc sensiblement non allergénique est constitué de caoutchouc synthétique, par exemple du caoutchouc de silicone ou un type de caoutchouc de toute autre composition.

5. Tapis selon l'une des revendications 1 ou 2, **caractérisé en ce que** le caoutchouc sensiblement non allergénique est un mélange de caoutchouc synthétique et de caoutchouc Para naturel.

6. Tapis selon la revendication 1 ou 2, **caractérisé en ce que** le caoutchouc sensiblement non allergénique est un tissu naturel ou synthétique.

7. Tapis selon l'une des revendications 1 ou 2, **caractérisé en ce que** le caoutchouc sensiblement non allergénique est un mélange de tissu naturel ou synthétique et de caoutchouc Para naturel.

8. Tapis selon l'une quelconque des revendications 1 à 7, **caractérisé en ce que** le matériau élastique pour le remplissage de la chambre (3) rembourrée entre la première et la deuxième couche (1, 2) est présent sous la forme de particules élastiques individuelles de forme sensiblement irrégulière ou est formé d'un autre matériau gélatineux ou poudreux ou aussi d'un matériau de consistance élastique particulière quelconque, ou ce dernier est mélangé également avec des particules élastiques selon des pourcentages différents et adéquats.

9. Tapis selon l'une des revendications 1 à 8, **caractérisé en ce que** le matériau élastique est constitué d'une substance naturelle ou synthétique, par exemple du caoutchouc Para ou du caoutchouc de silicone, ou d'un mélange de substances différentes.
